# EUROPEAN PATENT APPLICATION

(11) **EP 4 806 437 A1**
(43) Date of publication of application: **16.09.2026**
(21) Application number: 24888165.8
(22) Date of filing: 05.11.2024
(51) Int. Cl.: A61K 31/506, A61K 38/26, A61K 45/06, A61P 1/16

(54) **PHARMACEUTICAL COMPOSITION FOR PREVENTION OR TREATMENT OF NONALCOHOLIC FATTY LIVER DISEASE**

(30) Priority: 06.11.2023 KR 20230152136; 03.06.2024 KR 20240072704
(71) Applicant: Dong-A ST Co., Ltd., Dongdaemun-gu Seoul 02587 (KR)
(72) Inventor: KIM, Mi-Kyung, Suwon-si Gyeonggi-do 16505 (KR); KIM, Tae Hyoung, Seoul 05501 (KR); LEE, Su Jin, Yongin-si Gyeonggi-do 17073 (KR); JUNG, Il Hoon, Yongin-si Gyeonggi-do 17066 (KR); CHAE, Yu Na, Yongin-si Gyeonggi-do 16853 (KR)
(74) Representative: Gille Hrabal Partnerschaftsgesellschaft mbB Patentanwälte
(86) International application number: PCT/IB2024/060898
(87) International publication number: WO 2025/099562

(57) **Abstract**

The present invention relates to a pharmaceutical composition for the prevention or treatment of nonalcoholic fatty liver disease.

## Description

### Technical Field

The present invention relates to a pharmaceutical composition for prevention or treatment of nonalcoholic fatty liver disease, including a G protein-coupled receptor 119 (GPR119) agonist as an active ingredient.

### Background

Fatty liver is a pathological state in which triglycerides excessively accumulate in hepatocytes, and is medically defined as a state in which triglycerides occupy at least 5% of the liver weight. Fatty liver is classified into alcoholic and nonalcoholic fatty liver depending on whether fatty liver is caused by excessive alcohol intake. Nonalcoholic fatty liver disease (NAFLD) is a group of diseases that encompasses the patterns of all diseases, ranging from nonalcoholic fatty liver to steatohepatitis and liver cirrhosis. Simple nonalcoholic fatty liver is a disease in which only fat deposition in the liver is present and only fat deposition in liver tissue is elevated due to insulin resistance and the like, without hepatocyte damage and fibrosis findings. Nonalcoholic fatty liver may progress to nonalcoholic steatohepatitis (NASH), accompanied by fibrosis due to hepatocyte damage caused by an inflammatory reaction due to oxidative stress and the like.

In particular, among nonalcoholic fatty liver diseases, nonalcoholic steatohepatitis (NASH) may progress to cirrhosis and liver cancer that cause irreversible liver damage, and increases the mortality rate associated with liver disease as well as overall mortality (Hepatology, 2012(55):2005-2023). As a result, according to statistics from the United States, as of 2013, nonalcoholic steatohepatitis has been a second most common cause disease of liver transplantation after hepatitis C, and is expected to be a first cause disease of liver transplantation over hepatitis C after 2020, so there is an urgent need to develop a drug for preventing and treating the same (Clinical Gastroenterology and Hepatology, 2019(17):748-755).

### Detailed Description of the Invention

### Technical Problem

One object of the present invention is to provide a pharmaceutical composition for prevention or treatment of nonalcoholic fatty liver disease, including a compound represented by formula 1, a pharmaceutically acceptable salt thereof, optical isomers thereof, hydrates or solvates thereof, or mixtures thereof.

### Technical Solution

According to one embodiment of the present invention, a pharmaceutical composition for prevention or treatment of nonalcoholic fatty liver disease comprises a compound represented by formula 1 below, a pharmaceutically acceptable salt thereof, optical isomers thereof, hydrates or solvates thereof, or mixtures thereof; and a glucagon-like peptide-1 (GLP-1) receptor agonist (GLP-1RA).

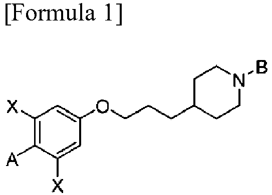

In above formula 1,
A is an oxadiazole group substituted with a C1-C6 linear or branched alkyl group,
B is a pyrimidine group substituted with a C1-C6 linear or branched alkyl group, and
X is each independently F, Cl, Br or I.

In one embodiment, A of above formula 1 may be 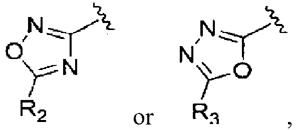 in which R₂ and R₃ may be each independently a C1-C6 linear or branched alkyl group.

In one embodiment, B of above formula 1 may be 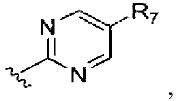 in which R₇ may be a C1-C6 linear or branched alkyl group.

In one embodiment, X of above formula 1 may be each F.

In one embodiment, the term "alkyl" may mean a linear or branched hydrocarbon residue, unless otherwise stated. Examples of the C1-C6 alkyl may include methyl, ethyl, propyl, isopropyl, butyl, isobutyl, pentyl, hexyl, and the like.

In one embodiment, the compound represented by above formula 1 may be 3-(4-(3-(1-(5-ethylpyrimidin-2-yl)piperidin-4-yl)propoxy)-2,6-difluorophenyl)-5-isopropyl-1,2,4-oxadiazole.

In one embodiment, the GLP-1 receptor agonist may include semaglutide, albiglutide, dulaglutide, exenatide, liraglutide, lixisenatide, orforglipron, danuglipron, or GSBR-1290, analogues thereof, or pharmaceutically acceptable salts thereof. Each of them may be used alone or in combination with two or more.

In the present invention, the term "analogue" may be used in the sense of including a fragment analogue of a peptide or a derivative thereof; or an analogue of a monomolecular compound or a derivative thereof.

In one embodiment, the GLP-1 receptor agonist may include semaglutide, albiglutide, dulaglutide, exenatide, liraglutide, lixisenatide, analogues thereof, or pharmaceutically acceptable salts thereof.

In one embodiment, the GLP-1 receptor agonist may include a GLP-1 analogue, an analogue thereof, or pharmaceutically acceptable salts thereof.

In one embodiment, the GLP-1 receptor agonist may include semaglutide, albiglutide, dulaglutide, liraglutide, analogues thereof, or pharmaceutically acceptable salts thereof.

In one embodiment, the GLP-1 receptor agonist may be semaglutide, an analogue thereof, or pharmaceutically acceptable salts thereof.

Semaglutide may be represented by formula 2 below, referred to as N-ε²⁶-[2-(2-{2-[2-(2-{2-[(S)-4-carboxy-4-(17-carboxyheptadecanoyl-amino)butyrylamino]ethoxy}ethoxy)acetylamino]ethoxy}ethoxy)acetyl][Aib⁸,Arg³⁴]GLP-1-(7-37), and prepared as described in Example 4 of WO2006/097537.

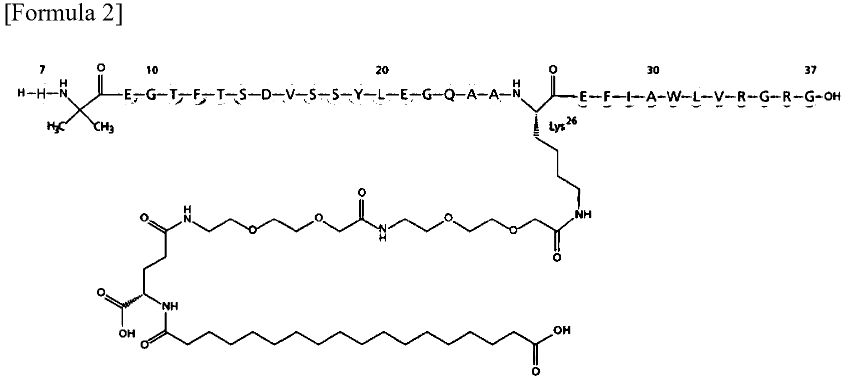

A pharmaceutically acceptable salt of semaglutide may be an acid addition salt or a base addition salt. Among the pharmaceutically acceptable salts of semaglutide, the acid addition salt may be, for example, hydrochloride, hydrobromide, hydroiodide, nitrate, sulfate, bisulfate, phosphate, acidic phosphate, isonicotinate, acetate, lactate, salicylate, citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate, butyrate, camphorate, camphorsulfonate, digluconate, glycerophosphate, hemisulfate, heptanoate, hexanoate, fumarate, hydrochloride, 2-hydroxyethanesulfonate (isethionate), nicotinate, 2-naphthalenesulfonate, oxalate, pectinate, persulfate, 3-phenylpropionate, picrate, pivalate, propionate, succinate, tartrate, thiocyanate, phosphate, glutamate, or bicarbonate.

The nonalcoholic fatty liver disease (NAFLD) may include nonalcoholic fatty liver, nonalcoholic steatohepatitis (NASH), liver fibrosis, or liver cirrhosis.

The nonalcoholic fatty liver disease may be nonalcoholic steatohepatitis.

The nonalcoholic steatohepatitis may be a disease accompanied by fibrosis due to an inflammatory reaction caused by oxidative stress or the like and hepatocyte damage caused thereby.

The nonalcoholic steatohepatitis may be a disease accompanied by fibrosis.

The pharmaceutical composition may be a pharmaceutical composition for prevention or treatment of nonalcoholic fatty liver disease which inhibits the deposition of triglycerides in liver tissue.

The pharmaceutical composition may be a pharmaceutical composition for prevention or treatment of nonalcoholic fatty liver disease which inhibits the occurrence of inflammation in liver tissue.

The pharmaceutical composition may be a pharmaceutical composition for prevention or treatment of nonalcoholic fatty liver disease which inhibits the fibrosis of liver tissue.

When it is described in the present specification that the pharmaceutical composition includes A and B, the pharmaceutical composition may be interpreted to mean a composition including A and B; a combination including A and B as separate agents, respectively; or a composition including the combination. Thus, in the present invention, the composition may be used interchangeably with the combination.

The pharmaceutical composition may include the compound represented by formula 1, the pharmaceutically acceptable salt thereof, optical isomers thereof, hydrates or solvates thereof, or mixtures thereof; and the GLP-1 receptor agonist, respectively, as a separate agent, or include all thereof as in form of a complex agent.

The pharmaceutical composition may be a combination of separate agents or a complex agent.

The pharmaceutical composition may include a first compartment containing a first active ingredient and a second compartment containing a second active ingredient. The first active ingredient may be the compound represented by formula 1, the pharmaceutically acceptable salt thereof, optical isomers thereof, hydrates or solvates thereof, or mixtures thereof. The second active ingredient may include the GLP-1 receptor agonist.

According to embodiments of the present invention, in the pharmaceutical composition, the first compartment containing the first active ingredient and the second compartment containing the second active ingredient may be co-administered, and the pharmaceutical composition may be a composition for co-administration. Specifically, the pharmaceutical composition may be a combination in which two ingredients are formulated in separate unit formulations. In this case, the first active ingredient and the second active ingredient may be co-administered in separate formulations.

When the pharmaceutical composition according to one embodiment is administered, it is confirmed that an NAFLD activity score (NAS) is remarkably reduced compared to a case of administering the compound represented by formula 1, the pharmaceutically acceptable salt thereof, optical isomers thereof, hydrates or solvates thereof, or mixtures thereof as a GPR119 agonist; or the GLP-1 receptor agonist, respectively.

When the pharmaceutical composition according to one embodiment is administered, it is confirmed that fat metabolism is remarkably improved in a synergistic manner in terms of a content of liver tissue cholesterol, a relative area of liver tissue fat, and a ratio of hepatocytes including lipid droplets (an image analysis index highly associated with steatosis score) compared to a case of administering the compound represented by formula 1, the pharmaceutically acceptable salt thereof, optical isomers thereof, hydrates or solvates thereof, or mixtures thereof as a GPR119 agonist; or the GLP-1 receptor agonist, respectively.

In the present invention, non-limiting examples of the pharmaceutically acceptable salt of the compound represented by above formula 1 may include a salt with an inorganic acid such as hydrochloric acid, bromic acid, phosphoric acid or sulfuric acid; a salt with an organic carboxylic acid such as acetic acid, trifluoroacetic acid, citric acid, maleic acid, oxalic acid, succinic acid, benzoic acid, tartaric acid, fumaric acid, mandelic acid, ascorbic acid or malic acid, or a sulfonic acid such as methanesulfonic acid or para-toluenesulfonic acid; a salt with an alkali metal such as sodium, potassium or lithium; a salt with various acids known to form other pharmaceutically acceptable salts; or the like.

The pharmaceutical composition for the prevention or treatment of nonalcoholic fatty liver disease, according to one embodiment, may be used in the form of a general pharmaceutical preparation. The pharmaceutical preparation may be administered in various oral and parenteral formulations at the time of administration, and the formulation may be variously determined according to a method of use.

When the pharmaceutical composition of one embodiment is formulated into various oral and parenteral formulations, it may be prepared using excipients such as commonly used fillers, diluents, extenders, binders, humectants, disintegrants, surfactants, and the like.

Solid preparations for oral administration may include tablets, pills, powders, granules, capsules, and the like, and such solid preparations may be prepared by mixing the pharmaceutical composition with at least one excipient, for example, starch, calcium carbonate, sucrose or lactose, gelatin, and the like. In addition, glidants such as magnesium stearate, talc, and the like may also be used in addition to simple excipients.

Furthermore, liquid preparations for oral administration may correspond to suspensions, liquids for internal use, emulsions, syrups, etc., but may also include various excipients, for example, humectants, sweeteners, fragrances, preservatives, etc., in addition to water and liquid paraffin, which are commonly used simple diluents.

Preparations for parenteral administration may include sterile aqueous solutions, non-aqueous solvents, suspensions, emulsions, lyophilized preparations, and suppositories. The non-aqueous solvents and the suspending solvents used herein may include propylene glycol, polyethylene glycol, vegetable oil like olive oil, injectable ester like ethyl oleate, etc. Bases of the suppositories used herein may include witepsol, macrogol, tween 61, cacao butter, laurinum, glycerinated gelatin, etc.

In addition, the pharmaceutical composition for prevention or treatment of nonalcoholic fatty liver disease according to the present invention may exhibit an effective amount in a dosage range of about 1 to about 1,000 mg. The amounts of administration or intake may be administered in various doses and methods, such being administered as once a day or several times a day by dividing the amount depending on a subject's weight, age, gender, health condition, diet, administration time, administration method, excretion rate, and severity of the disease.

In the present invention, the nonalcoholic fatty liver disease may include both primary and secondary nonalcoholic fatty liver diseases. Specifically, the nonalcoholic fatty liver disease in the present invention may include simple fatty liver, nonalcoholic steatohepatitis, and liver fibrosis and liver cirrhosis generated by the progression of the disease, but is not limited thereto.

In one embodiment, the nonalcoholic fatty liver disease (NAFLD) in the present invention may be substantially the same as the metabolic dysfunction-associated steatotic liver disease (MASLD) referred to in the United States, Europe, and the like.

In one embodiment, the nonalcoholic fatty liver disease (NAFLD) in the present invention may be substantially the same as the metabolic dysfunction-associated fatty liver disease (MAFLD).

In one embodiment, the nonalcoholic steatohepatitis (NASH) in the present invention may be substantially the same as the metabolic dysfunction associated steatohepatitis (MASH) referred to in the United States, Europe, and the like.

The present invention may also provide a method for preventing or treating nonalcoholic fatty liver disease, which includes administering a therapeutically effective amount of a pharmaceutical composition including the compound represented by formula 1, the pharmaceutically acceptable salt thereof, optical isomers thereof, hydrates or solvates thereof, or mixtures thereof; and the GLP-1 receptor agonist into a subject in need of treatment.

In the present specification, the term "subject in need of treatment" may mean a mammal, including humans, and the term "administration" may mean providing a certain substance to a subject by any suitable method. The term "therapeutically effective amount" may mean an amount of an active ingredient or a pharmaceutical composition that induces a biological or medical response in an animal or human being considered by a researcher, veterinarian, doctor, or other clinician, which includes an amount that induces symptomatic relief of the disease or disorder being treated. It may be obvious to those skilled in the art that a therapeutically effective dose and the number of administrations for the active ingredient of the present invention will vary depending on the desired effect.

In one embodiment, the term "administration route of the pharmaceutical composition of the present invention" may be administered through any general route as long as it may reach a target tissue.

It may be performed through oral administration, intraperitoneal administration, intravenous administration, intramuscular administration, subcutaneous administration, endothelial administration, intranasal administration, intrapulmonary administration, rectal administration, intraluminal administration, intraperitoneal administration, and intrathecal administration, but is not limited thereto.

The pharmaceutical composition of one embodiment may be administered once a day or twice or more a day at regular time intervals.

The pharmaceutical composition of one embodiment may be used alone or in combination with methods using surgery, hormone therapy, drug therapy, and a biological response modifier for the prevention and treatment of nonalcoholic fatty liver disease.

One embodiment may provide a composition for prevention or amelioration of nonalcoholic fatty liver disease, which includes the compound represented by above formula 1, the pharmaceutically acceptable salt thereof, optical isomers thereof, hydrates or solvates thereof, or mixtures thereof; and the GLP-1 receptor agonist as an active ingredient. The composition may be a food composition or a feed composition.

In one embodiment, the term "amelioration" may mean all the acts, by which a symptom of diseases gets better or takes a favorable turn by administering the composition.

In one embodiment, the term "food" may include meat, sausage, bread, chocolate, candy, snacks, confectionery, pizza, ramen, other noodles, gum, dairy products including ice cream, various soups, beverages, tea, drinks, alcoholic beverages, vitamin complexes, health functional foods, health foods, health supplements, and the like, and include all foods in a common sense. The term "health functional food" may be the same term as a food for special health use (FoSHU), and may refer to a food processed to efficiently exhibit biomodulation functions in addition to nutrition supply, thus providing high medical and medicinal effects. Here, by "function(ality)," it may be meant to control nutrients for the structure and function of the human body or obtain a useful effect for health purposes, such as physiological actions, etc. The term "health food" may mean a food having an active health maintenance or promotion effect compared to a general food, and the term "health supplement food" may refer to a food for the purpose of health supplements. In some cases, the terms of health functional food, health food, and health supplement food may be interchangeably used. The food of the present invention may be prepared by a method commonly used in the art, and may be prepared by adding raw materials and ingredients commonly added in the art during the preparation. Specifically, the food may include proteins, carbohydrates, fats, nutrients, seasoning agents, and flavoring agents, and examples of the carbohydrates may include glucose, fructose, maltose, sucrose, oligosaccharide, dextrin, cyclodextrin, xylitol, sorbitol, erythritol, saccharin, or a synthetic flavoring agent, but are not limited thereto. The food composition of the present invention may be prepared in various forms of formulations without limitation as long as it is a formulation recognized as a food.

In the present invention, the term "feed" may mean any natural or artificial diet, single meal, etc., or an ingredient of the single meal, so that the livestock may take in and digest or, which may be appropriate to do so. The feed may include a feed additive or a feed supplement. The kind of the feed may not be particularly limited, and may include any feed commonly used in the art. Non-limiting examples of the feed may include plant feeds such as grains, root fruits, food processing by-products, algae, fibers, pharmaceutical by-products, oils and fats, starches, gourds, grain by-products or the like; and animal feeds such as proteins, inorganics, oils and fats, minerals, single-cell proteins, animal planktons, food products, or the like. These may be used alone or in combination of two or more.

The present invention may also provide a method for preventing or ameliorating nonalcoholic fatty liver disease, which includes administering a food or feed composition including the compound represented by above formula 1, the pharmaceutically acceptable salt thereof, optical isomers thereof, hydrates or solvates thereof, or mixtures thereof; and a GLP-1 receptor agonist as an active ingredient into a subject in need of amelioration.

The present invention may also provide a use of the pharmaceutical composition including the compound represented by above formula 1, the pharmaceutically acceptable salt thereof, optical isomers thereof, hydrates or solvates thereof, or mixtures thereof; and the GLP-1 receptor agonist as an active ingredient for preventing or treating nonalcoholic fatty liver disease.

The present invention may also provide a use of the pharmaceutical composition including the compound represented by above formula 1, the pharmaceutically acceptable salt thereof, optical isomers thereof, hydrates or solvates thereof, or mixtures thereof; and the GLP-1 receptor agonist as an active ingredient for preparing a medication for prevention or treatment of nonalcoholic fatty liver disease.

Matters mentioned in each of the items of the pharmaceutical composition, treatment method, food composition, amelioration method, feed composition, and use according to embodiments of the present invention may be equally applied to each of the embodiments, if not contradictory to each other.
**(1)** The present invention provides a pharmaceutical composition for the prevention or treatment of nonalcoholic fatty liver disease, including a compound represented by formula 1 below, a pharmaceutically acceptable salt thereof, optical isomers thereof, hydrates or solvates thereof, or mixtures thereof; and a GLP-1 receptor agonist.

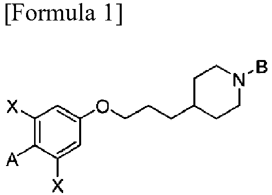

In above formula 1,
A is an oxadiazole group substituted with a C1-C6 linear or branched alkyl group,
B is a pyrimidine group substituted with a C1-C6 linear or branched alkyl group, and
X is each independently F, Cl, Br, or I.

**(2)** In above (1), the GLP-1 receptor agonist may include semaglutide, albiglutide, dulaglutide, exenatide, liraglutide, lixisenatide, orforglipron, danuglipron, GSBR-1290, analogues thereof, or pharmaceutically acceptable salts thereof.

**(3)** In above (1) or (2), the A may be 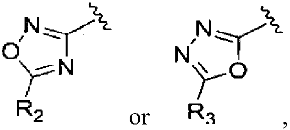 and R₂ and R₃ may be each independently a C1-C6 linear or branched alkyl group.

**(4)** In any one of above (1) to (3), the B may be 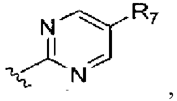 and R₇ may be a C1-C6 linear or branched alkyl group.

**(5)** In any one of above (1) to (4), the compound represented by above formula 1 may be 3-(4-(3-(1-(5-ethylpyrimidin-2-yl)piperidin-4-yl)propoxy)-2,6-difluorophenyl)-5-isopropyl-1,2,4-oxadiazole.

**(6)** In any one of above (1) to (5), the GLP-1 receptor agonist may include semaglutide, albiglutide, dulaglutide, exenatide, liraglutide, lixisenatide, analogues thereof, or pharmaceutically acceptable salts thereof.

**(7)** In any one of above (1) to (6), the GLP-1 receptor agonist may include semaglutide, an analogue thereof, or pharmaceutically acceptable salts thereof.

**(8)** In any one of above (1) to (7), the nonalcoholic fatty liver disease may include at least any one selected from the group consisting of simple fatty liver, nonalcoholic steatohepatitis, liver fibrosis, and liver cirrhosis.

**(9)** In any one of above (1) to (8), the pharmaceutical composition may inhibit fibrosis of liver tissue.

**(10)** The present invention provides a method for preventing or treating nonalcoholic fatty liver disease, the method including administering a pharmaceutical composition including a compound represented by formula 1 below, a pharmaceutically acceptable salt thereof, optical isomers thereof, hydrates or solvates thereof, or mixtures thereof; and a GLP-1 receptor agonist.

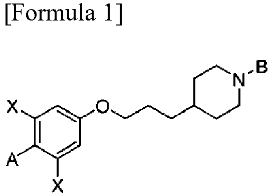

In above formula 1,
A is an oxadiazole group substituted with a C1-C6 linear or branched alkyl group,
B is a pyrimidine group substituted with a C1-C6 linear or branched alkyl group, and
X is each independently F, Cl, Br, or I.

**(11)** The composition according to above (10) may be the composition according to any one of above (1) to (9).

**(12)** In above (10) or (11), the method may include administering the compound represented by formula 1, the pharmaceutically acceptable salt thereof, optical isomers thereof, hydrates or solvates thereof, or mixtures thereof; and administering the GLP-1 receptor agonist.

**(13)** In above (12), the GLP-1 receptor agonist may be administered after administering the compound represented by formula 1, the pharmaceutically acceptable salt thereof, optical isomers thereof, hydrates or solvates thereof, or mixtures thereof.

**(14)** In above (12), the compound represented by formula 1, the pharmaceutically acceptable salt thereof, optical isomers thereof, hydrates or solvates thereof, or mixtures thereof are administered after administering the GLP-1 receptor agonist.

**(15)** The present invention provides a use of a pharmaceutical composition for preventing or treating nonalcoholic fatty liver disease, including a compound represented by formula 1 below, a pharmaceutically acceptable salt thereof, optical isomers thereof, hydrates or solvates thereof, or mixtures thereof; and a GLP-1 receptor agonist.

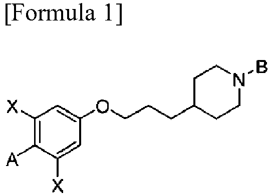

In above formula 1,
A is an oxadiazole group substituted with a C1-C6 linear or branched alkyl group,
B is a pyrimidine group substituted with a C1-C6 linear or branched alkyl group, and
X is each independently F, Cl, Br, or I.

**(16)** In above (15), the pharmaceutical composition may be the composition according to any one of above (1) to (9).

**(17)** The present invention provides a use of a pharmaceutical composition for preparing a medication for preventing or treating nonalcoholic fatty liver disease, including a compound represented by formula 1 below, a pharmaceutically acceptable salt thereof, optical isomers thereof, hydrates or solvates thereof, or mixtures thereof; and a GLP-1 receptor agonist.

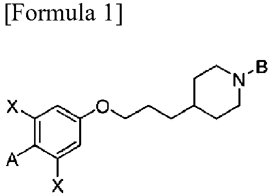

In above formula 1,
A is an oxadiazole group substituted with a C1-C6 linear or branched alkyl group,
B is a pyrimidine group substituted with a C1-C6 linear or branched alkyl group, and
X is each independently F, Cl, Br, or I.

**(18)** In above (17), the pharmaceutical composition may be the composition according to any one of above (1) to (9).

**(19)** The present invention provides a pharmaceutical combination including a compound represented by above formula 1, a pharmaceutically acceptable salt thereof, optical isomers thereof, hydrates or solvates thereof, or mixtures thereof; and a GLP-1 receptor agonist.

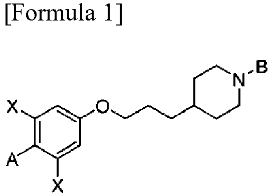

In above formula 1,
A is an oxadiazole group substituted with a C1-C6 linear or branched alkyl group,
B is a pyrimidine group substituted with a C1-C6 linear or branched alkyl group, and
X is each independently F, Cl, Br, or I.

**(20)** In above (19), the GLP-1 receptor agonist may include semaglutide, albiglutide, dulaglutide, exenatide, liraglutide, lixisenatide, orforglipron, danuglipron, GSBR-1290, analogues thereof, or pharmaceutically acceptable salts thereof.

**(21)** In above (19) or (20), the A may be 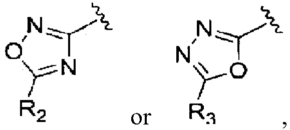 and R₂ and R₃ may be each independently a C1-C6 linear or branched alkyl group.

**(22)** In any one of above (19) to (21), the B may be 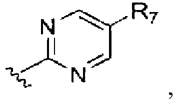 and R₇ may be a C1-C6 linear or branched alkyl group.

**(23)** In any one of above (19) to (22), the compound represented by above formula 1 may be 3-(4-(3-(1-(5-ethylpyrimidin-2-yl)piperidin-4-yl)propoxy)-2,6-difluorophenyl)-5-isopropyl-1,2,4-oxadiazole.

**(24)** In any one of above (19) to (23), the GLP-1 receptor agonist may include semaglutide, albiglutide, dulaglutide, exenatide, liraglutide, lixisenatide, analogues thereof, or pharmaceutically acceptable salts thereof.

**(25)** In any one of above (19) to (24), the GLP-1 receptor agonist may include semaglutide, an analogue thereof, or pharmaceutically acceptable salts thereof.

**(26)** In any one of above (19) to (25), the combination may be for preventing or treating nonalcoholic fatty liver disease.

**(27)** The present invention provides a pharmaceutical composition for prevention or treatment of nonalcoholic fatty liver disease, including a compound represented by formula 1 below, a pharmaceutically acceptable salt thereof, optical isomers thereof, hydrates or solvates thereof, or mixtures thereof, which are used in combination with a GLP-1 receptor agonist.

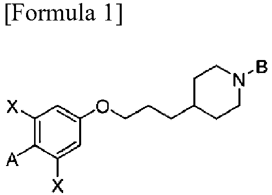

In above formula 1,
A is an oxadiazole group substituted with a C1-C6 linear or branched alkyl group,
B is a pyrimidine group substituted with a C1-C6 linear or branched alkyl group, and
X is each independently F, Cl, Br, or I.

**(28)** The present invention provides a pharmaceutical composition for prevention or treatment of nonalcoholic fatty liver disease, including a GLP-1 receptor agonist, which is used in combination with a compound represented by formula 1 below, a pharmaceutically acceptable salt thereof, optical isomers thereof, hydrates or solvates thereof, or mixtures thereof.

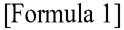

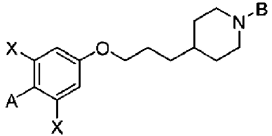

In above formula 1,
A is an oxadiazole group substituted with a C1-C6 linear or branched alkyl group,
B is a pyrimidine group substituted with a C1-C6 linear or branched alkyl group, and
X is each independently F, Cl, Br, or I.

**(29)** In above (27) or (28), the compound represented by formula 1, the pharmaceutically acceptable salt thereof, optical isomers thereof, hydrates or solvates thereof, or mixtures thereof; and the GLP-1 receptor agonist may correspond to any one of above (2) to (9).

### Advantageous Effects

The pharmaceutical composition according to the present invention can exhibit excellent effects in preventing or treating nonalcoholic fatty liver disease.

### Brief Description of Drawings

FIGS. 1 and 2 are views showing NAS results in DIO-NASH mice.
FIGS. 3 to 5 are views showing lipid metabolism results in the liver of DIO-NASH mice.
FIGS. 6 to 8 are views showing the results of fibrosis in a liver fibrosis mouse model.
FIGS. 9 and 10 are views showing the results of the expression of fibrosis genes in the liver fibrosis mouse model.

### Mode for Invention

The pharmaceutical composition according to the present invention may include the compound represented by above formula 1, the pharmaceutically acceptable salt thereof, optical isomers thereof, hydrates or solvates thereof, or mixtures thereof; and the GLP-1 receptor agonist as an active ingredient. The pharmaceutical composition may exhibit an excellent effect of treating or preventing nonalcoholic fatty liver disease by a complementary effect of the above-described active ingredients.

The pharmaceutical composition of one embodiment may exhibit a synergistic effect on the treatment or prevention of nonalcoholic fatty liver disease compared to the case of individually administering each of the above-described active ingredients.

In the present specification, "at least one" may be interpreted to indicate all combinations derived from a plurality of configurations. For example, "at least one of A, B, and C" may be interpreted to include "A, B, or C," "two selected from A, B, and C," and all combinations derived from "A, B, and C."

GPR119 may be one of the G protein-coupled receptors (GPCR) in which Gs is coupled to activate signaling through cAMP. GPR119 may have a high expression level in pancreatic beta cells and L cells of the small intestine, may also be present in the liver, and may be a regulator for the biosynthesis of fatty acids occurring in the liver. In addition, the activation of GP119 may suppress the differentiation and activation of inflammatory cells and suppress the activation of hepatic stellate cells, thereby controlling all of fatty liver, inflammation, and fibrosis in nonalcoholic fatty liver disease.

Hereinafter, the pharmaceutical composition according to one embodiment of the present invention will be described in detail with reference to Examples and Comparative Examples.

Hereinafter, compound 1 mean 3-(4-(3-(1-(5-ethylpyrimidin-2-yl)piperidin-4-yl)propoxy)-2,6-difluorophenyl)-5-isopropyl-1,2,4-oxadiazole.

Hereinafter, "GLP-1RA" described in the experimental result is a GLP-1 receptor agonist, which means semaglutide.

### Efficacy in improving NAFLD activity score (NAS) in DIO-NASH mice

Five-week-old male C57BL/6J mice were fed with a special diet (Research Diet, D09100301) containing high fat, high fructose, and high cholesterol for 36 weeks, thereby inducing nonalcoholic steatohepatitis. After that, compound 1 (oral) and semaglutide (subcutaneous injection) were administered to DIO-NASH mice at 100 mg/kg and 30 nmol/kg, respectively, for eight weeks, once a day, alone or in combination, and then NAFLD activity scores (NAS) before/after dosing were compared through liver tissue biopsy. The NAS was analyzed as follows.

Liver tissue separated through biopsy and necropsy was fixed in 10% neutral buffered formalin and prepared into a paraffin block to obtain a tissue section having a thickness of 3 µm. After that, a hematoxylin and eosin (HE) stain was performed, and the ratio of deposited lipid droplets in the stained liver tissue, the degree of inflammatory cell infiltration, and the degree of cell necrosis were classified into three types, i.e., steatosis, inflammation, and hepatocyte ballooning, as histological features, and expressed as the NAS.

The NAS criteria are shown in Table 1 below.

**[Table 1]**

| **Histological features** | **Score (Grade)** | **Definition** |
|---|---|---|
| Fat accumulation (Steatosis) | 0 | No fat |
| | 1 | <33% of the hepatic parenchyma |
| | 2 | 34-66% of the hepatic parenchyma |
| | 3 | >66% of the hepatic parenchyma |
| Inflammation | 0 | None |
| | 1 | 1-2 foci per 200 x field |
| | 2 | 3-4 foci per 200 x field |
| | 3 | More than 4 foci per 200 x field |
| Hepatocyte ballooning | 0 | None |
| | 1 | Few balloon cells |
| | 2 | Many cells/prominent ballooning |

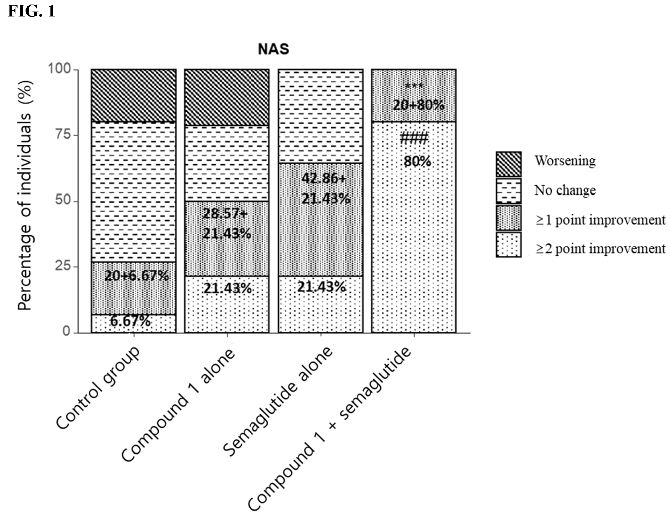

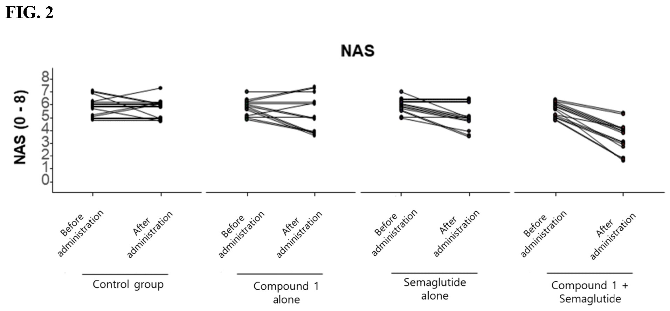

Comparing the NAS before/after administration, animals whose NAS after dosing is improved (reduced) by at least two points (≥2 points improvement) compared to the NAS before dosing, animals whose NAS after dosing is improved (reduced) by at least one point (≥1 point improvement), animals whose NAS is unchanged (no change), and animals whose NAS score after dosing is increased (worsens) are shown in FIG. 1 as a percentage with regard to the total number of animals. The NAS before/after dosing is shown in FIG. 2. An analysis was performed according to one-sided Fisher's exact test with Bonferroni correction, and *** means P<0.001 compared to the vehicle (control) for animals with at least one point improvement, and ### means P<0.001 compared to the vehicle (control) for animals with at least two point improvement (***: P < 0.001 compared to vehicle for ≥1 point improvement. ###: P < 0.001 compared to vehicle for ≥2 point improvement). The animals with at least one point improvement are the sum of the animals with one point improvement in NAS and the animals with at least two points of improvement in NAS (the number of animals in each group is n=14-15).

Referring to FIGS. 1 and 2, it can be confirmed that the NAS was reduced by at least one point compared to before dosing in the animals (100%) of the co-administered group with compound 1 and semaglutide together, and thus, symptoms took a favorable turn in all the animals. It can even be confirmed that 80% of all animals had NAS improved by at least two points. It can be confirmed that in the co-administered group of animals, at least two points were improved in at least one item of fat accumulation, hepatocyte expansion, and inflammation; or at least one point was improved in at least two items thereof.

On the contrary, it can be confirmed that animals with at least two points of improvement accounted for 6.67% in the control group, 21.43% in the group dosed with compound 1 alone, and only 21.43% in the group dosed with semaglutide alone, and it was confirmed that the results were about four times better upon the co-administration.

### Efficacy in improving lipid metabolism in the liver of DIO-NASH mice

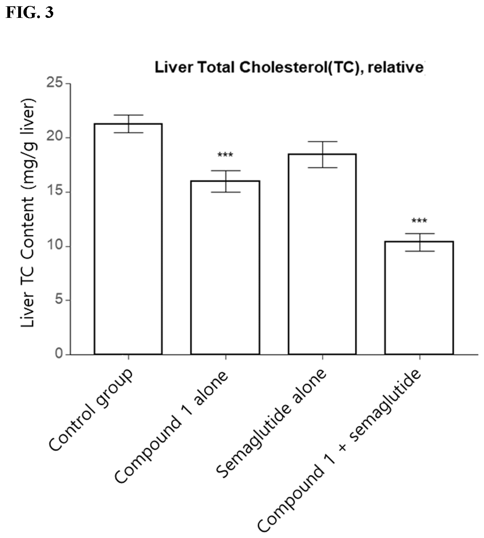

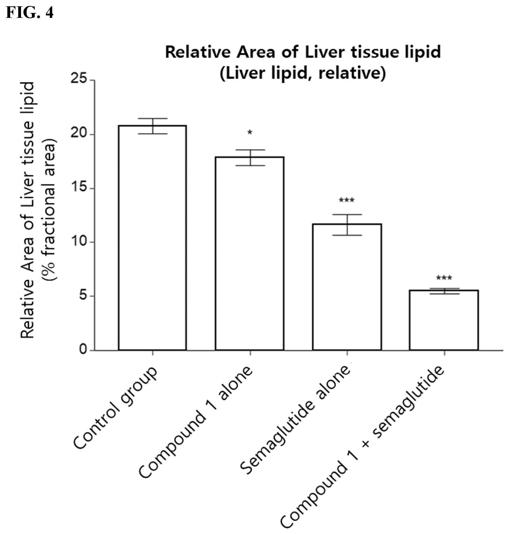

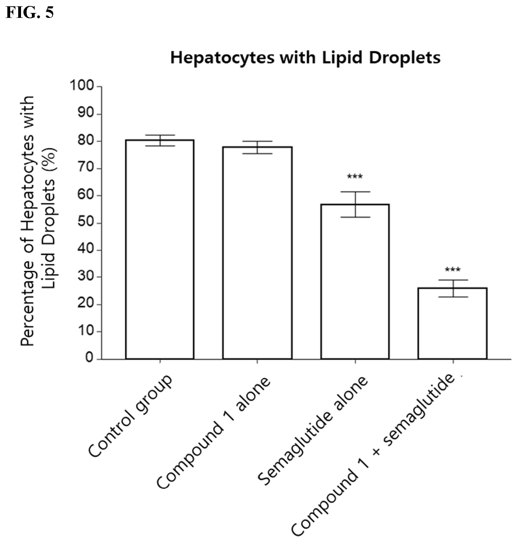

After administering the most effective dose of compound 1 and semaglutide alone or in combination to the DIO-NASH mice once a day for eight weeks, liver tissue biopsy was performed to analyze the content of liver tissue cholesterol, the relative area of liver tissue lipid, and the ratio of hepatocytes including lipid droplets (an image analysis index highly associated with steatosis score). The content of liver tissue cholesterol was measured using a commercially available kit of an automatic analyzer after cholesterol extraction, and the relative area of liver tissue lipid was quantified by image analysis of an HE staining slide. The results are shown in FIGS. 3, 4, and 5, respectively. In FIGS. 3 to 5, values are expressed as the mean + SEM for the number of animals in each group n=14-15 (values expressed as a mean of n=14-15 + SEM) and were analyzed according to Dunnett's test one-factor linear model, and *** indicates P<0.001 compared to the vehicle (control group).

Referring to FIGS. 3 to 5, it can be confirmed that when compound 1 and semaglutide were administered together, fat metabolism was synergistically improved compared to each single administration.

### Efficacy in ameliorating the fibrosis in the liver fibrosis mouse model

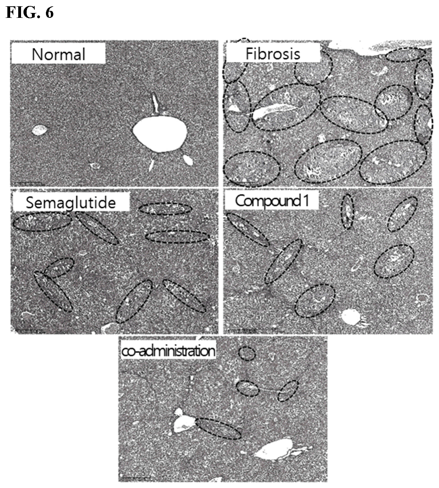

Compound 1 was orally administered once a day for four weeks after inducing liver fibrosis with GAN feed and CCl₄, and semaglutide was subcutaneously injected twice a week. Each thereof was administered at 100 mg/kg and 250 nmol/kg. After staining with Masson's trichrome (MT), the stained fibrosis area was quantified. In addition, a change in liver weight was also measured. The results are shown in FIGS. 6, 7, and 8. In FIGS. 6 to 8, values are expressed as the mean + SEM for the normal group n=4 and the number of animals in each group n=11-18 (values expressed as a mean of n=11-18 (NASH-fibrosis) + SEM) and were analyzed according to the T-test or Dunnett's test, and ** indicates P<0.01 compared to the vehicle (control group). In FIGS. 6 to 8, "normal" is a group of normal mice, "fibrosis" is a control group of fibrosis, "semaglutide" or "GLP1RA" is a group dosed with semaglutide alone, "compound 1" is a group dosed with compound 1 alone, and "co-administration" is a group dosed with semaglutide and compound 1 in combination.

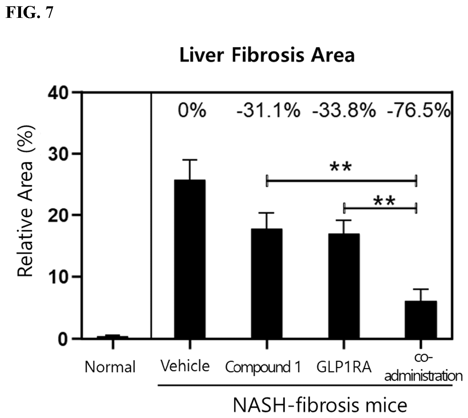

In FIG. 6, regions including a fibrosis area are indicated by dotted ellipses so that the fibrosis area may be identified separately from other regions. The quantification result of FIG. 7 is a result of quantifying only the fibrosis area.

Referring to FIGS. 6 and 7, it can be confirmed that fibrosis is remarkably increased in the control group of fibrosis compared to the group of normal mice, and it can be confirmed that relatively less fibrosis is shown in the group dosed with semaglutide or compound 1 alone compared to the control group of fibrosis. Compared with these, the synergistic therapeutic effect of decreasing fibrosis was confirmed in the co-administered group.

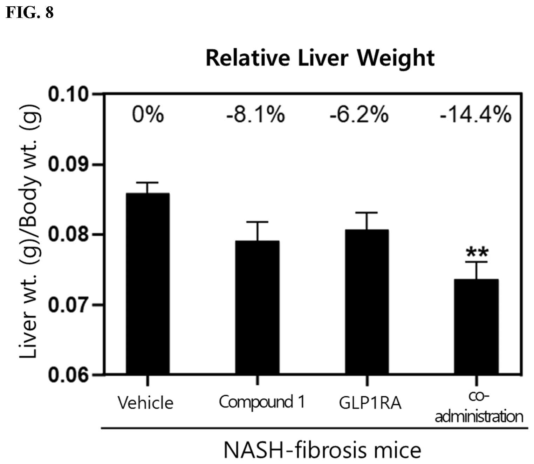

Referring to FIG. 8, it can be confirmed that a change in relative liver weight also showed a synergistic decrease upon co-administration compared to the group dosed with compound 1 or semaglutide alone.

### Inhibitory effect on fibrosis gene expression in the liver fibrosis mouse model

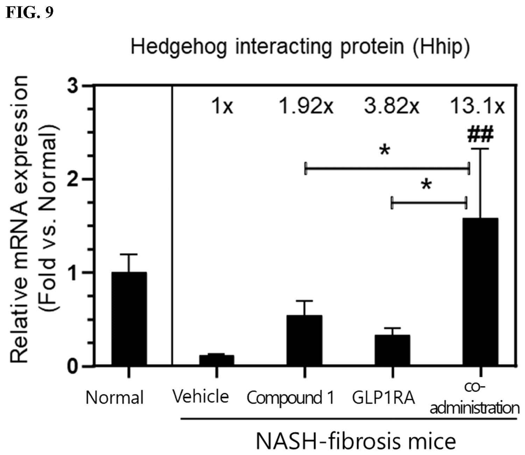

An expression of mRNA in hedgehog interacting protein (*Hhip*), which inhibits the activation of hepatic stellate cells, was evaluated. The results are shown in FIG. 9. In FIG. 9, values are expressed as the mean + SEM for the normal group of animals n=4 and the number of animals in each group n=11-18 (values expressed as a mean of n=11-18 (NASH-fibrosis) + SEM) and were analyzed according to the T-test or Dunnett's test, and ** indicates P<0.05 between two groups. ## indicates P<0.01 compared to vehicle (control group).

Referring to FIG. 9, the expression of mRNA was reduced in the fibrotic mice (vehicle (control)) compared to normal mice, and the expression of mRNA was increased compared to the vehicle (control) by administration of compound 1 or semaglutide alone. Meanwile, it was confirmed that the expression of mRNA is remarkably increased above the normal level by the co-administration thereof. This means that the co-administration of compound 1 and semaglutide shows a synergistic effect of inhibiting the activation of hepatic stellate cells.

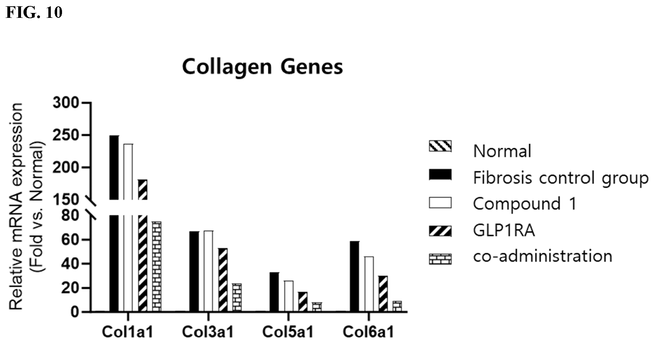

An expression of collagen genes in liver tissue was evaluated by a reverse transcription polymerase chain reaction. Ribonucleic acid (RNA) was extracted from liver tissue with a TRIzol reagent to separate the same, and complementary DNA (cDNA) was synthesized using reverse transcriptase, and then the expression of fibrosis-related genes (*Col1a1*, *Col3a1*, *Col5a1*, *Col6a1*) was analyzed by a real-time polymerase chain reaction. The results are shown in FIG. 10 and Table 2 below.

**[Table 2]**

| Percent reduction (unit, %) vs. fibrosis control group | Compound 1 | GLP1RA | Co-administration |
|---|---|---|---|
| *Col1a1* | -5% | -27% | -70% |
| *Col3a1* | 0% | -21% | -64% |
| *Col5a1* | -20% | -48% | -75% |
| *Col6a1* | -21% | -48% | -84% |

Referring to FIG. 10 and Table 2, it can be confirmed that an expression of all four types of collagen was increased in the control group of fibrosis compared to the normal group, and a percent reduction was synergistically increased upon co-administration compared to a single administration. This demonstrates the effect of reducing the expression of fibrosis genes by inhibiting the activation of hepatic stellate cells.

## Claims

1. A pharmaceutical composition for prevention or treatment of nonalcoholic fatty liver disease, comprising a compound represented by formula 1 below, a pharmaceutically acceptable salt thereof, optical isomers thereof, hydrates or solvates thereof, or mixtures thereof; and a GLP-1 receptor agonist: 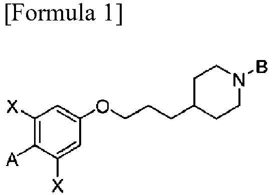
wherein in above formula 1,
A is an oxadiazole group substituted with a C1-C6 linear or branched alkyl group,
B is a pyrimidine group substituted with a C1-C6 linear or branched alkyl group, and
X is each independently F, Cl, Br, or I.

2. The pharmaceutical composition for prevention or treatment of nonalcoholic fatty liver disease of claim 1, wherein the GLP-1 receptor agonist comprises semaglutide, albiglutide, dulaglutide, exenatide, liraglutide, lixisenatide, orforglipron, danuglipron, GSBR-1290, analogues thereof, or pharmaceutically acceptable salts thereof.

3. The pharmaceutical composition for prevention or treatment of nonalcoholic fatty liver disease of claim 1, wherein the A is 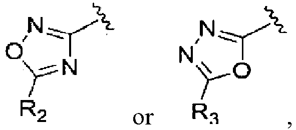 and R₂ and R₃ are each independently a C1-C6 linear or branched alkyl group.

4. The pharmaceutical composition for prevention or treatment of nonalcoholic fatty liver disease of claim 1, wherein the B is 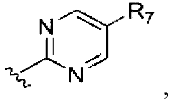 and R₇ is a C1-C6 linear or branched alkyl group.

5. The pharmaceutical composition for prevention or treatment of nonalcoholic fatty liver disease of claim 1, wherein the compound represented by above formula 1 is 3-(4-(3-(1-(5-ethylpyrimidin-2-yl)piperidin-4-yl)propoxy)-2,6-difluorophenyl)-5-isopropyl-1,2,4-oxadiazole.

6. The pharmaceutical composition for prevention or treatment of nonalcoholic fatty liver disease of claim 1, wherein the GLP-1 receptor agonist comprises semaglutide, albiglutide, dulaglutide, exenatide, liraglutide, lixisenatide, analogues thereof, or pharmaceutically acceptable salts thereof.

7. The pharmaceutical composition for prevention or treatment of nonalcoholic fatty liver disease of claim 1, wherein the GLP-1 receptor agonist comprises semaglutide, an analogue thereof, or pharmaceutically acceptable salts thereof.

8. The pharmaceutical composition for prevention or treatment of nonalcoholic fatty liver disease of claim 1, wherein nonalcoholic fatty liver disease comprises at least one selected from the group consisting of simple fatty liver, nonalcoholic steatohepatitis, liver fibrosis, and liver cirrhosis.

9. The pharmaceutical composition for prevention or treatment of nonalcoholic fatty liver disease of claim 1, wherein the pharmaceutical composition inhibits fibrosis of liver tissue.

10. A method for preventing or treating nonalcoholic fatty liver disease, comprising administering the pharmaceutical composition according to claim 1.

11. The method for preventing or treating nonalcoholic fatty liver disease of claim 10, the method comprising:
administering the compound represented by formula 1, the pharmaceutically acceptable salt thereof, optical isomers thereof, hydrates or solvates thereof, or mixtures thereof; and
administering the GLP-1 receptor agonist.

12. A use of the pharmaceutical composition according to claim 1 for treating or preventing nonalcoholic fatty liver disease.

13. A use of the pharmaceutical composition according to claim 1 for preparing a medication for treatment or prevention of nonalcoholic fatty liver disease.

14. A pharmaceutical combination comprising the compound represented by formula 1 below, the pharmaceutically acceptable salt thereof, optical isomers thereof, hydrates or solvates thereof, or mixtures thereof; and the GLP-1 receptor agonist: 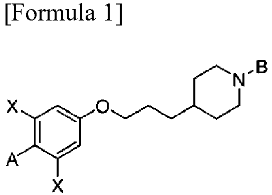
wherein in above formula 1,
A is an oxadiazole group substituted with a C1-C6 linear or branched alkyl group,
B is a pyrimidine group substituted with a C1-C6 linear or branched alkyl group, and
X is each independently F, Cl, Br, or I.

15. The pharmaceutical combination of claim 14, wherein the combination is for preventing or treating nonalcoholic fatty liver disease.
